# EUROPÄISCHE PATENTANMELDUNG

(11) **EP 3 915 590 A1**
(43) Veröffentlichungstag der Anmeldung: **01.12.2021**
(21) Anmeldenummer: 21176351.1
(22) Anmeldetag: 27.05.2021
(51) Int. Cl.: A61L 2/00, A61L 2/06

(54) **VORRICHTUNG UND VERFAHREN ZUM STERILISIEREN DURCH KNALL-GASREAKTON MITTELS EINER FLAMME**

(30) Priorität: 27.05.2020 DE 102020114221
(71) Anmelder: Syntegon Technology GmbH, 71332 Waiblingen (DE)
(72) Erfinder: Eisenschmid, Ralph, 71672 Marbach am Neckar (DE)
(74) Vertreter: DREISS Patentanwälte PartG mbB

(57) **Zusammenfassung**

Die Erfindung betrifft eine Vorrichtung (10) zur Entpyrogenisierung und zum Dekontaminieren eines Packmittels (20).

## Beschreibung

Die vorliegende Erfindung betrifft eine Vorrichtung und Verfahren zum schlagartigen Sterilisieren eines Packmittels. Ein Packmittel kann bspw. ein Behälter oder eine Flasche oder ähnliches sein. Das Packmittel kann aus Kunststoff, Glass oder ähnlichem sein.

Bekannt sind Sterilisationsbehandlungen mit Heißluft (für einige Minuten bei einer Temperatur über 300 °C) und einer anschließenden Abkühlungsbehandlung. Die so behandelten Packmittel/Behälter werden anschließend verschlossen. Dieses Vorgehen ist mit hohem Energieverbrauch verbunden.

Was durch das Aufheizen des gesamten Packmittels bedingt ist.

DE 10 2014 100 375 A1 beschreibt die Verwendung einer Düse mittels derer eine knallgasbasierte Flamme erzeugbar ist. Die Düse wird in den Innenraum eines flaschenförmigen Behälters bewegt und dort zum "kontinuierlichen Abbrennen" von Verunreinigungen verwendet.

DE 101 34 037 A1 zeigt eine Reinigungseinrichtung in der zu sterilisierende Gegenstände durch eine Plasma-Reaktionszone bewegt werden.

Der vorliegenden Erfindung liegt die Aufgabe zugrunde, eine Möglichkeit zur schnellen und energieeffizienten Sterilisation und Entpyrogenisierung von Packmitteln bereitzustellen, wobei im Vorliegenden mit Entpyrogenisierung eine Totaloxidation von Verunreinigungen bildenden organischen Substanzen gemeint ist.

Diese Aufgabe wird durch eine Vorrichtung nach Anspruch 1 und ein Verfahren nach Anspruch 7 gelöst.

Die erfindungsgemäße Vorrichtung zur Entpyrogenisierung und Dekontamination bzw. Sterilisation eines Packmittels, umfasst eine Dekontaminationskammer, also eine geschlossene Kammer bzw. einen Raum, in dem der Dekontaminationsvorgang durchgeführt wird. Sie umfasst weiter eine Fördereinrichtung, die ausgebildet ist, um das Packmittel der Dekontaminationskammer zuzuführen und/oder aus der Dekontaminationskammer zu entnehmen. Mittels der Fördereinrichtung kann das Packmittel also in die bzw. aus der Dekontaminationskammer bewegt werden. Die Dekontaminationskammer kann eine Schleuseneinrichtung zum Zuführen des Packmittels und/oder Entnehmen des Packmittels aufweisen. Die Schleuseneinrichtung kann klappenartig ausgebildet sein, andere Arten an Schleuseneinrichtungen sind ebenso möglich. Die Schleuseneinrichtung erlaubt ein Öffnen und Schließen des die Dekontaminationskammer bildenden Hohlraums. Die Vorrichtung umfasst weiter eine Einrichtung zum Zuführen eines Reinigungsreaktionsgases in die Dekontaminationskammer. Das Reinigungsreaktionsgas dient zur Durchführung der eigentlichen Entpyrogenisierung bzw. der eigentlichen Sterilisation bzw. Dekontamination in der Dekontaminationskammer. Die Vorrichtung ist dabei ausgebildet, so dass eine schlagartige Reinigungsreaktion durchgeführt werden kann. Die Reinigungsrektion läuft also nicht kontinuierlich ab, sondern wird in der geschlossenen Dekontaminationskammer einmalig ausgelöst, läuft ab und ist dann beendet. Weiter umfasst die Vorrichtung eine Auslösevorrichtung zum Auslösen einer Reinigungsreaktion des Reinigungsreaktionsgases in der Dekontaminationskammer. Weiter umfasst die Vorrichtung eine Einrichtung zum Abführen des Reinigungsreaktionsgases aus der Dekontaminationskammer nach Ablauf der Reinigungsreaktion, also wenn die Reinigungsreaktion beendet ist.

Die Dekontaminationskammer ist im abgeschlossenen Zustand also vor Auslösen der Reinigungsreaktion typischerweise im Wesentlichen, insbesondere vollständig, gasdicht geschlossen. Das Reinigungsreaktionsgas ist typischerweise ein Knallgasgemisch, also ein Gemisch aus Wasserstoff und Sauerstoff. Das Reinigungsreaktionsgas kann weitere Komponenten umfassen. Bspw. kann das Reinigungsreaktionsgas ein Inertgas, insbesondere Stickstoff, umfassen. Die Zugabe von Inertgas kann die Stärke der Reinigungsreaktion moderieren bzw. einstellen. Das Reinigungsreaktionsgas kann auch ein Gas enthalten, das bei Anregung UV-Strahlung emittiert, bspw. kann das Reinigungsreaktionsgas Xenon und/oder Argon enthalten.

Das erfindungsgemäße Verfahren zum Reinigen eines Packmittels umfasst die Schritte:
Einbringen eines Packmittels in eine
Dekontaminationskammer;
optional evakuieren der Dekontaminationskammer;
Erzeugen einer Reinigungsreaktionsgas enthaltenden Atmosphäre in der Dekontaminationskammer;
Auslösen einer schlagartig ablaufenden Reinigungsreaktion des Reinigungsreaktionsgases in der Dekontaminationskammer;
Abführen der Reaktionsprodukte der Reinigungsreaktion aus der Dekontaminationskammer nachdem die Reinigungsreaktion beendet ist;
optional spülen der Dekontaminationskammer mit einem Spülgas;
optional evakuieren der Dekontaminationskammer;
Entnehmen des Packmittels aus der Dekontaminationskammer;
Das Verfahren kann mittels der erfindungsgemäßen Vorrichtung durchgeführt werden. Vorteile und Weiterbildungsmöglichkeiten des Verfahrens sind als ebenso in Bezug auf die Vorrichtung beschrieben zu verstehen und umgekehrt sind Vorteile und Weiterbildungsmöglichkeiten der Vorrichtung als ebenso in Bezug auf das Verfahren beschrieben zu verstehen.

Im Gegensatz zu bekannten Vorgehensweisen wird bei dem erfindungsgemäßen Verfahren bzw. durch die erfindungsgemäße Vorrichtung sichergestellt, dass das gesamte Packmittel bzw. dessen gesamte Oberfläche (Innenseite und Außenseite) der Reinigungsreaktion ausgesetzt ist und nicht nur bspw. eine Innenseite. Gegenüber Heißluftsterilisationsprozessen kann das Erfindungsgemäße Verfahren wesentlich schneller durchgeführt werden, da zur Reinigung nicht erst das Packmittel, welches bspw. aus Glas bestehen kann, erhitzt werden muss. Beim erfindungsgemäßen Verfahren (bzw. bei Verwendung der Vorrichtung) werden die Packmittel nur oberflächlich einer Erhitzung ausgesetzt und dies auch nur für sehr kurze Zeit. Insgesamt gesehen, also den Herstellungsprozess des Reinigungsreaktionsgases miteingeschlossen, kann das erfindungsgemäße Verfahren auch mit niedrigerem Energieaufwand und daher niedrigeren Kosten als die bekannten Verfahren durchgeführt werden. Die hohen Temperaturen bei der Reinigungsreaktion, insbesondere bei Verwendung von Wasserstoff und Sauerstoffgemischen, führen auch zu einer guten Pyrolyse organischer Verunreinigungen. Hierzu kann insbesondere mit gegenüber dem Wasserstoff stöchiometrischem Sauerstoffüberschuss gearbeitet werden. Der Sauerstoff kann je nach Grad der Verschmutzung mit organischen Verunreinigungen angepasst werden. Bei stärkerer organischer Verschmutzung wird also zusätzlicher Sauerstoff bereitgestellt, um die Verschmutzungen pyrolytisch zu zersetzen bzw. zu CO2 bzw. CO zu verbrennen.

Insbesondere ist die Dekontaminationskammer im Gegensatz zu bekannten Verfahren bei Durchführung der Reinigungsreaktion vollständig geschlossen.

Die Auslöseeinrichtung löst insbesondere nicht kontinuierliche die Reinigungsreaktion aus, sondern zeitlich punktuell also an einzelnen bestimmten Zeitpunkten, woraufhin die Reaktion dann abläuft. Insbesondere führt die Einrichtung zum Abführen des Reinigungsreaktionsgases die Reaktionsprodukte erst nach Ablauf der Reinigungsreaktion ab. Im Stand der Technik hingegen sind kontinuierliche Prozesse bekannt in denen eine kontante Reaktion lokal begrenzt am Laufen gehalten wird und zu sterilisierende Gegenstände durch die Reaktionszone bewegt werden.

Die Fördereinrichtung kann eine Fördereinheit umfassen, die ausgebildet ist, um das Packmittel der Dekontaminationskammer zuzuführen und ebenfalls aus der Dekontaminationskammer zu entnehmen. Bspw. kann ein durch die Dekontaminationskammer laufendes Band vorgesehen sein. Die Dekontaminationskammer kann auf die Oberfläche des Bandes auf der das Packmittel angeordnet ist, abgesenkt werden und mit der Oberfläche des Bandes gasdicht abschließen. In diesem Fall wäre die offene Seite der Dekontaminationskammer im Zusammenspiel mit der Oberfläche des Bandes als Schleuseneinrichtung zu sehen.

Die Fördereinrichtung kann auch eine erste Fördereinheit umfassen, die ausgebildet ist, um das Packmittel der Dekontaminationskammer zuzuführen und eine zweite Fördereinheit umfassen, die ausgebildet ist, um das Packmittel aus der Dekontaminationskammer zu entnehmen. Zubringen und Abführen des Packmittels kann also mittels unterschiedlicher Fördereinheiten realisiert werden.

Die Fördereinheit, die erste Fördereinheit oder zweite Fördereinheit können jeweils ein Förderband zum Fördern des Packmittels und/oder eine Greifereinheit zum Greifen und Bewegen des Packmittels umfassen. Bspw. kann das jeweilige Band rasch Packmittel fördern und die Greifereinheit diese in die Dekontaminationskammer einsetzen. Andere Arten von Fördereinheiten sind ebenso im Sinne der Erfindung.

Die Auslösevorrichtung zum Auslösen einer Reinigungsreaktion kann ausgebildet sein, um eine Knall-Gasreaktion mittels einer Flamme oder eines, insbesondere elektrisch erzeugten, Funkens auszulösen. Die Auslösevorrichtung kann bspw. einen Lichtbogen zwischen zwei Drahtspitzen, an denen eine Spannung anliegt, erzeugen. Möglich ist auch die Auslösung mittels Mikrowellen, eines Piezozünders und/oder einer stillen elektrischen Entladung (Dielectric barrier discharge). Die stille elektrische Entladung (auch dielektrische Barriereentladung, DBE, englisch Dielectric Barrier Discharge, DBD) oder Plasmaentladung ist eine Wechselspannungs-Gasentladung, bei der mindestens eine der Elektroden vom Gasraum durch galvanische Trennung mittels eines Dielektrikums elektrisch isoliert ist.

Die Vorrichtung kann eine Evakuierungsvorrichtung umfassen, mittels der die Dekontaminationskammer evakuierbar ist. Die Evakuierungsvorrichtung kann also verwendet werden, um die Dekontaminationskammer vor dem Befüllen mit dem Reaktionsgas zu evakuieren, so dass die Atmosphäre in der Dekontaminationskammer möglichst kontrolliert erzeugt werden kann. Im Anschluss an die Reinigungsreaktion kann mittels der Evakuierungsvorrichtung die Dekontaminationskammer evakuiert werden, um Verbrennungsprodukte bzw. verbleibende Gase der Atmosphäre in der Dekontaminationskammer zu entfernen. Insbesondere bei Flaschenartigen Packmitteln kann eine Evakuierung einen Austausch des die Atmosphäre in der Dekontaminationskammer bildenden Gases beschleunigen, da ein Flascheninnenraum ansonsten einem Gasaustausch nur langsam zugänglich sein kann. Die Packmittel im Sinne der Erfindung können insbesondere flaschenförmig sein, einen verjüngten Flaschenhals aufweisen und einen bauchigen Flaschenkörper. Die Packmittel können insbesondere unter Verwendung von Glas hergestellt sein oder aus Glas bestehen. Die Anwendung des erfindungsgemäßen Verfahrens ist hier besonders energiesparend, da auf ein Aufheizten des thermisch trägen Glasmaterials verzichtet wird.

Die Vorrichtung kann eine Kühleinrichtung umfassen. Die Kühleinrichtung kann aktive Kühlung für die Wände der Dekontaminationskammer bereitstellen. Bspw. kann die Kühleinrichtung ausgebildet sein, um Kühlmittel bspw. entlang von Kühlkanälen entlang der Wände der Dekontaminationskammer zu leiten. Die Kühleinrichtung kann auch ausgebildet sein, um eine passive Kühlung für die Dekontaminationskammer bzw. deren Wände bereitzustellen. Es kann bspw. vorgesehen sein, dass Kühlrippen an den Wänden bzw. and deren Außenseite der Dekontaminationskammer angebracht sind. Jedenfalls sind spezielle Maßnahmen zur Kühlung der Wände der Dekontaminationskammer vorgesehen.

Die Dekontaminationskammer ist also typischerweise von Wänden eingeschlossen, die die Dekontaminationskammer größtenteils, insbesondere vollständig umschließen bzw. begrenzen. Diese Wände können einer Kühlung bedürfen, da das Durchführen der Reinigungsreaktion Prozesswärme freisetzt und diese, je nach Verfahrensführung und Ausbildung der Wände der Dekontaminationskammer, abgeführt werden kann/sollte. Dies erfolgt unter anderem über die Kühleinrichtung.

Die Vorrichtung kann eine Gasmischeinheit umfassen, die ausgebildet ist, um das Reinigungsreaktionsgas in seiner vorgesehenen Zusammensetzung aus einzelnen Komponenten zu mischen. Bspw. kann die Gasmischeinheit Wasserstoff, Sauerstoff und ein oder mehrere Inertgase in vorgesehenem Verhältnis mischen bevor das Reinigungsreaktionsgas über die Einrichtung zum Zuführen eines Reinigungsreaktionsgases in die Dekontaminationskammer überführt wird.

Bei dem Verfahren kann das Reinigungsreaktionsgas eine oxidierende Atmosphäre bilden, also bspw. einen Sauerstoffüberschuss aufweisen. Beim Auslösen der Reinigungsreaktion des Reinigungsreaktionsgases in der Dekontaminationskammer ist entsprechend überschüssiger Sauerstoff vorhanden, welcher mit kontaminierenden Substanzen oxidierend reagieren kann. Bei dem Verfahren kann das Reinigungsreaktionsgas auch eine reduziedierende Atmosphäre bilden, also bspw. einen Sauerstoffmangel aufweisen. Beim Auslösen der Reinigungsreaktion des Reinigungsreaktionsgases in der Dekontaminationskammer ist entsprechend weniger Sauerstoff als Reaktionspartner (bspw. Wasserstoff) vorhanden. Eine Verfahrensführung mit reduziedierender Atmosphäre kann bspw. dazu dienen Oxidschichten zu entfernen.

Bei dem Verfahren kann das Reinigungsreaktionsgas Wasserstoff und Sauerstoff umfassen. Wasserstoff und Sauerstoff bilden sogenanntes Knallgas. Das Gemisch der beiden Gase reagiert nach dem Auslösen bzw. Zünden bei sehr hoher Temperatur. Das Gemisch der beiden Gase reagiert auch ohne potentiell verschmutzende Komponenten zu hinterlassen, da das Reaktionsprodukt Wasser bzw., durch die hohe Energiefreisetzung bei der Reaktion, Wasserdampf ist. Der Reinigungsvorgang basiert quasi auf einer Verpuffungsreaktion und läuft instantan ab. Die Reaktion kann auch als Explosion oder Detonation geführt werden.

Bei dem Verfahren kann das Reinigungsreaktionsgas aus Wasserstoff und Sauerstoff, optional einem Inertgas, insbesondere Stickstoff, und optional einem bei Anregung UV-Strahlung emittierenden Gas, insbesondere Xenon und/oder Argon, bestehen. Die Zugabe von Inertgas kann die Stärke der Reinigungsreaktion moderieren bzw. einstellen. Ein bei Anregung UV-Strahlung emittierenden Gas kann durch die Reaktion von Sauerstoff und Wasserstoff angeregt werden und entsprechend UV-Strahlung abgeben. Die abgegebene UV-Strahlung kann einen weiteren Reinigungseffekt haben.

Es kann vorgesehen sein, dass in der Dekontaminationskammer eine Vorrichtung zur Bewirkung einer Plasmaentladung in die heißen Reaktionsgase vorgesehen ist. Hiermit können bspw. die UV-Strahlung emittierenden Gase zur Emmision von UV-Strahlung angeregt werden.

Die Vorrichtung kann weiter ein in der Dekontaminationskammer angeordnetes entkeimungsverstärkendes Strahlmittel umfasses, das insbesondere als UV-Leuchtmittel, insbesondere Blitzlampe, Quecksilberdampflampe, Xenon-Excimer-Lampe und/oder UV-LED Leuchtmittel ausgebildet sein kann.

Bei dem Verfahren kann das Reinigungsreaktionsgas in der Dekontaminationskammer vor Auslösen der Reinigungsreaktion auf einen Druck gebracht werden, der höher als der Atmosphärendruck ist. Hierdurch kann mehr Energie bei der Reinigungsreaktion freigesetzt werden. Es kann der Reinigungsprozess intensiviert werden.

Bei dem Verfahren kann das Reinigungsreaktionsgas in der Dekontaminationskammer vor Auslösen der Reinigungsreaktion auf einen Druck gebracht werden, der niedriger als der Atmosphärendruck ist. Hierdurch kann weniger Energie bei der Reinigungsreaktion freigesetzt werden. Es kann der Reinigungsprozess mit geringerer Intensivität durchgeführt werden.

Bisher wurde überwiegend auf die Entfernung organischer Verschmutzungen mittels der erfindungsgemäßen Vorrichtung bzw. dem Verfahren eingegegangen. Die bei der Reinigung ablaufenden Reaktionen können auch (alternativ oder zusätzlich) genutzt werden, um Oberflächen der zu reinigenenden Objekte bzw. Packmittel zu aktivieren. Eine derartige Aktivierung kann bspw. in der Hydrophilisierung ursprünglich hydrophober Kunststoffe liegen, die Benetzbarkeit der Objekte kann also modifiziert werden. Die bei der Reinigung ablaufenden Reaktionen können auch (alternativ oder zusätzlich) genutzt werden, um diffusionsgetriebene Vorgänge an Oberflächen der behandelten Objekte kontrolliert zu beschleunigen, bspw. Dotierung von Halbleitern und/oder metallischen Dünnfilmlegierungen.

Bei dem Verfahren kann vorgesehen sein, dass die Reinigungsreaktion des Reinigungsreaktionsgases mehrmals hintereinander durchgeführt wird, bevor die Packmittel die Dekontaminationskammer verlassen, dabei wird die Dekontaminationskammer typischerweise zwischen den Reinigungsreaktionen gespült.

Die erfindungsgemäße Vorrichtung bzw. das Verfahren können auch zur thermischen Vorsilikonisierung (Oberflächenbehandlung) von Glasobjekten, bspw. von Glas Vials eingesetzt werden.

Bei der Silikonisierung von Glas für Arzneimittel wird eine dünne Silikonschicht an der Innenwand des Glasbehälters aufgebracht. Das Silikon wirkt durch seine hydrophoben Eigenschaften wie eine Barriere zwischen der Glaswand und dem Füllprodukt und verhindert so, dass die empfindlichen Pharmazeutika am Glas haften. Die Glasbehälter lassen sich durch die wasserabweisende Silikonschicht rückstandsfrei entleeren. Vor allem bei hochwertigen Pharmazeutika ist dies relevant. Die Silikonisierung von Vials ist technisch Anspruchsvoll. Bei Spritzen oder Karpulen wird das Silikon typischerweise lediglich als Gleitfläche auf nur wenigen Stellen der Innenoberfläche aufgetragen. Bei Vials sollte die gesamte Innenoberfläche gleichmäßig silikonisiert werden.

Bei der Silikonisierung von Objekten, insbesondere Vials, wird in diese eine silikonhaltige Emulsion gesprüht. Danach wird die Emulsion in der Dekontaminationskammer mit mit wenigstens einer Knallgasreaktion getrocknet.

Die Erfindung wird im Folgenden anhand der Figuren näher beschrieben, wobei gleiche oder funktional gleiche Elemente ggf. lediglich einmal mit Bezugszeichen versehen sind. Die Beschreibung dient als Beispiel und ist nicht einschränkend zu verstehen.

Es zeigen:
- Figur 1: eine erfindungsgemäße Vorrichtung; und
- Figur 2: eine alternative erfindungsgemäße Vorrichtung.

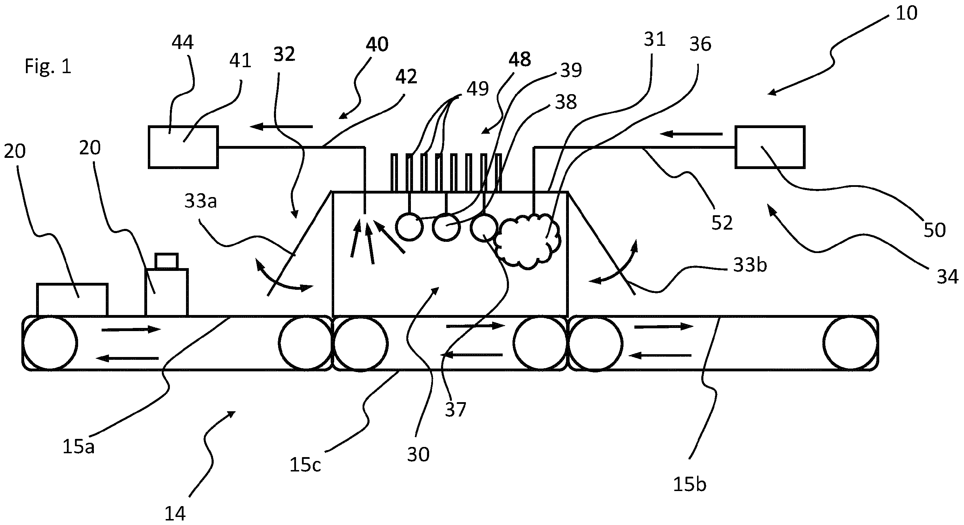

Fig. 1 zeigt eine Vorrichtung 10 zur Entpyrogenisierung und Dekontaminieren eines Packmittels 20. Im Beispiel von Figur 1 sind zwei unterschiedliche Packmittel 20 gezeigt. Das links dargestellte Packmittel 20 ist ein unter Verwendung von Kunststoff hergestellter im Wesentlichen rechteckig ausgebildeter Container. Das weiter rechts dargestellte Packmittel 20 ist ein flaschenartig ausgebildeter und vorliegend aus Glas bestehender Behälter.

Die Vorrichtung 10 umfasst eine Dekontaminationskammer 30. die Packmittel 20 können mittels einer Fördereinrichtung 14, die eine erste Fördereinheit 15a umfasst, der Dekontaminationskammer 30 zugeführt werden. Nach Abschluss der Behandlung in der Dekontaminationskammer 30 (die Behandlung wird später noch im Detail erläutert) können die Packmittel 20 mittels einer zweiten Fördereinheit 15b aus der Dekontaminationskammer 30 abgeführt bzw. hinausbewegt werden. Die erste Fördereinheit 15a und die zweite Fördereinheit 15b sind jeweils als umlaufende Bänder ausgebildet. In der Dekontaminationskammer 30 können die Packmittel 20 mittels einer dritten Fördereinheit 15c bewegt werden.

Die Vorrichtung 10 weist eine Schleuseneinrichtung 32 zum Zuführen des Packmittels 20 und Entnehmen des Packmittels 20 aus der Dekontaminationskammer 30 auf. Die Schleuseneinrichtung 32 umfasst hierzu eine erste Schleuseneinheit 33a, die als eine öffen- und schließbare Klappe in einer Wand 31 bzw. einem Gehäuse 31 der Dekontaminationskammer 30 ausgebildet ist und dem Zuführen der Packmittel 20 in die Dekontaminationskammer 30 dient. Die Schleuseneinrichtung 32 weist auch eine zweite Schleuseneinheit 33b auf, die ähnlich der ersten Schleuseneinheit 33a ausgebildet ist und dem Entnehmen der Packmittel 20 aus der Dekontaminationskammer 30 dient.

Die Vorrichtung 10 umfasst weiter eine Einrichtung 34 zum Zuführen eines Reinigungsreaktionsgases 36, dass vorliegend schematisch dargestellt ist, in die Dekontaminationskammer 30. Das Reinigungsreaktionsgases 36 ist im vorliegenden Beispiel eine Mischung aus Wasserstoff und Sauerstoff, optional einem Inertgas, insbesondere Stickstoff, und optional einem bei Anregung UV-Strahlung emittierenden Gas, insbesondere Xenon und/oder Argon.

Die Vorrichtung 10 umfasst weiter eine Gasmischeinheit 50, die vorliegend ein Teil der Einrichtung 34 zum Zuführen eines Reinigungsreaktionsgases 36 ist. Die Gasmischeinheit 50 ist ausgebildet, um das Reinigungsreaktionsgas 36 in seiner vorgesehenen Zusammensetzung aus einzelnen Komponenten zu mischen. Über eine Zuführleitung 52 Teil der Einrichtung 34 Gasgemisch der Dekontaminationskammer 30 zugeführt.

Die Vorrichtung 10 umfasst weiter ein entkeimungsverstärkendes Strahlmittel 37, das insbesondere als UV-Leuchtmittel, insbesondere Blitzlampe, Quecksilberdampflampe, Xenon-Excimer-Lampe und/oder UV-LED Leuchtmittel ausgebildet sein kann.

Die Vorrichtung 10 umfasst weiter eine Auslösevorrichtung 38 zum Auslösen einer Reinigungsreaktion des Reinigungsreaktionsgases 36 in der Dekontaminationskammer 30. Vorliegend ist die Auslösevorrichtung 38 ausgebildet, um einen Zündfunken abzugeben, der das Reinigungsreaktionsgas 36, das Wasserstoff und Sauerstoff enthält, entzünden kann. Die resultierende Reinigungsreaktion (Reaktion Wasserstoff und Sauerstoff) erzeugt hohe Temperaturen und tötet damit Keime ab, organische Verschmutzungen werden pyrolytisch zersetzt. Hierzu kann eine stöchiometrischer Sauerstoff Überschuss vorgesehen sein.

Die Vorrichtung 10 umfasst weiter eine Vorrichtung 39 zur Bewirkung einer Plasmaentladung in die heißen Reaktionsgase nach Auslösen der Reinigungsreaktion des Reinigungsreaktionsgases 36 Hiermit können bspw. UV-Strahlung emittierenden Gase im Reinigungsreaktionsgas 36 zur Emmision von UV-Strahlung angeregt werden.

Die Vorrichtung 10 umfasst weiter eine Einrichtung 40 zum Abführen des Reinigungsreaktionsgases 36 aus der Dekontaminationskammer 30 nach Ablauf der Reinigungsreaktion. Die Einrichtung 40 zum Abführen der Reaktionsprodukte des Reinigungsreaktionsgases 36 umfasst eine Absaugeinrichtung 41, die über eine Absaugleitung 42 mit der Dekontaminationskammer 30 verbunden ist. Die Absaugeinrichtung 41 kann als Evakuierungsvorrichtung 44 ausgebildet sein. Hierzu kann die Absaugeinrichtung 41 eine entsprechend hohe Saugleistung aufweisen, sodass sie beispielsweise den Druck in der Dekontaminationskammer 30 auf unter 0,3bar, insbesondere 0,1 bar, insbesondere unter 0,05 bar absenken kann.

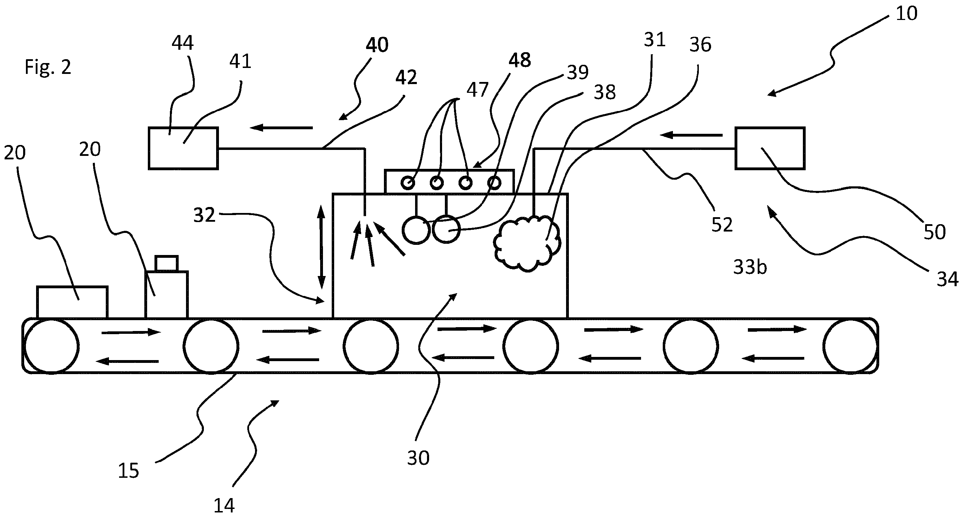

Die Vorrichtung kann eine Kühleinrichtung 48 umfassen. Die Kühleinrichtung 48 kann, wie in Fig. 1 illustriert, ausgebildet sein, um eine passive Kühlung für die Dekontaminationskammer 30 bzw. deren Wände 31 bzw. Gehäuse bereitzustellen. Es kann bspw. vorgesehen sein, dass Kühlrippen 49 an den Wänden 31 der Dekontaminationskammer 30 bzw. and deren Außenseite angebracht sind. Die Kühleinrichtung 48 kann auch aktive Kühlung für die Wände 31 der Dekontaminationskammer 30 bereitstellen. Bspw. kann die Kühleinrichtung 48 ausgebildet sein, um Kühlmittel bspw. entlang von Kühlkanälen 47 entlang der Wände 31 der Dekontaminationskammer 30 zu leiten. Eine derartige Ausbildung ist in Figur 2 gezeigt. Jedenfalls könne als Kühleinrichtung 48 spezielle Maßnahmen zur Kühlung der Wände 31 der Dekontaminationskammer 30 vorgesehen sein.

Im Beispiel von Fig. 2 ist auch vorgesehen, dass das Gehäuse 31 auf eine Fördereinheit 15, die die Packmittel 20 sowohl in die Dekontaminationskammer 30 hinein und auch hinausbewegen kann, absenkbar ist bzw. von dieser abhebbar ist. Das Gehäuse 31 der Dekontaminationskammer 30 bildet mit der Oberfläche der Fördereinheit 15 gemeinsam eine Schleuseneinrichtung 32.

Die Kühleinrichtungen 48 der Beispiele der Figuren 1 und 2 kann jeweils bei der Ausführungsart gemäß der anderen Figur vorgesehen sein. Die Fördereinrichtung 14 der Beispiele der Figuren 1 und 2 kann jeweils bei der Ausführungsart gemäß der anderen Figur vorgesehen sein. Die Schleuseneinrichtung 32 der Beispiele der Figuren 1 und 2 kann jeweils bei der Ausführungsart gemäß der anderen Figur vorgesehen sein.

## Patentansprüche

1. Vorrichtung (10) zum schlagartigen Entpyrogenisierung und Dekontaminieren eines Packmittels (20), umfassend:
eine Dekontaminationskammer (30), eine Fördereinrichtung (14), die ausgebildet ist, um das Packmittel (20) der Dekontaminationskammer (30) zuzuführen und/oder aus der Dekontaminationskammer (30) abzuführen, **dadurch gekennzeichnet, dass** die Vorrichtung (10) eine Schleuseneinrichtung (32) zum Zuführen des Packmittels (20) und/oder Entnehmen des Packmittels (20) aufweist, dass die Vorrichtung (10) eine Einrichtung (34) zum Zuführen eines Reinigungsreaktionsgases (36) in die Dekontaminationskammer (30) aufweist, dass die Vorrichtung (10) eine Auslösevorrichtung (38) zum Auslösen einer Reinigungsreaktion des Reinigungsreaktionsgases (36) in der Dekontaminationskammer (30) aufweist, und dass die Vorrichtung (10) eine Einrichtung (40) zum Abführen des Reinigungsreaktionsgases (36) aus der Dekontaminationskammer (30) nach Ablauf der Reinigungsreaktion aufweist.

2. Vorrichtung (10) nach Anspruch 1, wobei die Fördereinrichtung (14), eine Fördereinheit (15) umfasst, die ausgebildet ist, um das Packmittel (20) der Dekontaminationskammer (30) zuzuführen und ebenfalls aus der Dekontaminationskammer (30) zu entnehmen oder wobei die Fördereinrichtung (14), eine erste Fördereinheit (15a) umfasst, die ausgebildet ist, um das Packmittel (20) der Dekontaminationskammer (30) zuzuführen und eine zweite Fördereinheit (15b) umfasst, die von der ersten Fördereinheit (15a) separat ausgebildet ist und weiter ausgebildet ist, um das Packmittel (20) aus der Dekontaminationskammer (30) zu entnehmen.

3. Vorrichtung (10) nach einem oder mehreren der vorigen Ansprüche, wobei die Auslösevorrichtung (38) zum Auslösen einer Reinigungsreaktion zum Auslösen einer Knall-Gasreaktion mittels einer Flamme, eines insbesondere elektrisch erzeugten, Funkens, Mikrowellen, eines Piezozünders und/oder einer stillen elektrischen Entladung (Dielectric barrier discharge).

4. Vorrichtung (10) nach einem oder mehreren der vorigen Ansprüche, wobei die Vorrichtung (10) eine Evakuierungsvorrichtung (44) umfasst, mittels der die Dekontaminationskammer (30) evakuierbar ist.

5. Vorrichtung (10) nach einem oder mehreren der vorigen Ansprüche, wobei die Vorrichtung (10) eine Kühleinrichtung (48) für die Wände der Dekontaminationskammer (30) umfasst.

6. Vorrichtung (10) nach einem oder mehreren der vorigen Ansprüche, wobei die Vorrichtung (10) eine Gasmischeinheit (50) umfasst, die ausgebildet ist, um das Reinigungsreaktionsgas (36) in seiner vorgesehenen Zusammensetzung aus einzelnen Komponenten zu mischen.

7. Verfahren zum schlagartigen Reinigen eines Packmittels (20) wobei das Verfahren die Schritte umfasst:
Einbringen eines Packmittels (20) in eine Dekontaminationskammer (30);
optional evakuieren der Dekontaminationskammer (30);
Erzeugen einer Reinigungsreaktionsgas (36) enthaltenden Atmosphäre in der Dekontaminationskammer (30) ;
Auslösen einer schlagartig ablaufenden Reinigungsreaktion des Reinigungsreaktionsgases (36) in der Dekontaminationskammer (30);
Abführen der Reaktionsprodukte der Reinigungsreaktion aus der Dekontaminationskammer (30) nach abgeschlossener Reinigungsreaktion;
optional spülen der Dekontaminationskammer (30) mit einem Spülgas;
optional evakuieren der Dekontaminationskammer (30);
Entnehmen des Packmittels (20) aus der Dekontaminationskammer (30).

8. Verfahren nach Anspruch 7, wobei das Reinigungsreaktionsgas (36) aus Wasserstoff und Sauerstoff, optional einem Inertgas, insbesondere Stickstoff, und optional einem bei Anregung UV-Strahlung emittierenden Gas, insbesondere Xenon und/oder Argon, besteht.

9. Verfahren nach einem der Ansprüche 7 bis 8, wobei das Reinigungsreaktionsgas (36) in der Dekontaminationskammer (30) vor Auslösen der Reinigungsreaktion auf einen Druck gebracht wird, der höher als der Atmosphärendruck ist.

10. Verfahren nach einem der Ansprüche 7 bis 9, wobei das Reinigungsreaktionsgas (36) in der Dekontaminationskammer (30) vor Auslösen der Reinigungsreaktion auf einen Druck gebracht wird, der niedriger als der Atmosphärendruck ist.
